# EUROPEAN PATENT APPLICATION

(11) **EP 3 239 290 A1**
(43) Date of publication of application: **01.11.2017**
(21) Application number: 15873020.0
(22) Date of filing: 21.12.2015
(51) Int. Cl.: C12M 3/00, C12M 1/00

(54) **CELL CULTURE APPARATUS AND CELL CULTURE BAG**

(30) Priority: 25.12.2014 JP 2014261531
(71) Applicant: OLYMPUS CORPORATION, Hachioji-shi Tokyo 192-8507 (JP)
(72) Inventor: KAWANO, Yoshihiro, Tokyo 192-8507 (JP); KOJIMA, Kiyotsugu, Tokyo 192-8507 (JP)
(74) Representative: Schicker, Silvia
(86) International application number: PCT/JP2015/085719
(87) International publication number: WO 2016/104452

(57) **Abstract**

A cell culture apparatus (100) is provided with: a cell culture bag (1) that is formed of a tubular membrane material that can accommodate cells and a culture medium therein and that has openings (4, 5) at both ends in the longitudinal direction, and that forms a flow passage for the culture medium from one opening (4) toward the other opening (5) in the longitudinal direction of the tubular membrane material; a solution supply means (2) that is connected to the one opening (4) and that supplies a solution to the inside of the cell culture bag (1); and a solution discharge means (3) that is connected to the other opening (5) and that discharges the solution from the inside of the cell culture bag (1).

## Description

### {Technical Field}

The present invention relates to a cell culture bag and a cell culture apparatus using the cell culture bag.

### {Background Art}

In recent years, with the progress of stem-cell research and regenerative medicine, there is a demand to prepare a large amount of cells for clinical use. When a large amount of cells are cultured, a sac-like culture bag that is formed of a material having gas permeability has been increasingly used instead of a flask or a petri dish (for example, see PTL 1) .

### {Citation List}

### {Patent Literature}

{PTL 1} Japanese Unexamined Patent Application, Publication No. 2000-125848

### {Summary of Invention}

### {Technical Problem}

During culturing, cells take in components required for growth, such as oxygen and nutrients, and discharge carbon dioxide and waste products. Therefore, in long-term cell culturing, because the culture medium deteriorates, the culture medium needs to be periodically replaced. However, replacing the culture medium requires troublesome work by a worker as well as the costs, and there is a possibility that the cell culture system is contaminated by bacteria etc. Therefore, there is a demand to perform the culture-medium replacement work as easily as possible.

The present invention has been made in view of the above-described circumstances, and an object thereof is to provide: a cell culture apparatus capable of easily performing culture-medium replacement and cell passaging in culturing using a cell culture bag; as well as a cell culture bag used in the apparatus.

### {Solution to Problem}

In order to achieve the above-described object, the present invention provides the following solutions.

According to one aspect, the present invention provides a cell culture apparatus including: a cell culture bag that is formed of a tubular membrane material that can accommodate cells and a culture medium therein and that has openings at both ends in the longitudinal direction, and that forms a flow passage for the culture medium, from one opening toward the other opening in the longitudinal direction of the tubular membrane material; a solution supply means that is connected to the one opening and that supplies a solution to the inside of the cell culture bag; and a solution discharge means that is connected to the other opening and that discharges the solution from the inside of the cell culture bag.

According to this aspect, a worker can easily perform the culture-medium replacement work during the culture period. Furthermore, because the cell culture bag forms the flow passage, it is possible to increase the cell adhesion area in a limited space and to increase the amount of cells that can be cultured.

The above-described aspect may further include: an accommodation vessel that accommodates at least part of the cell culture bag and that can hold the solution therein; an accommodation-vessel solution supply means that supplies the solution to the accommodation vessel; and an accommodation-vessel solution discharge means that discharges the solution from the accommodation vessel, wherein the at least part of the cell culture bag is formed of a porous membrane.

Accordingly, it is possible to easily perform culture-medium replacement and cell passaging.

In the above-described aspect, the solution discharge means may be provided with a solution suction means that suctions the solution from the inside of the cell culture bag.

In the above-described aspect, the accommodation-vessel solution discharge means may be provided with a solution suction means that suctions the solution from the inside of the accommodation vessel.

Accordingly, the worker operates the solution suction means, thereby making it possible to easily perform culture-medium replacement and cell passaging.

In the above-described aspect, the solution supply means may be provided with a solution delivery means that pumps the solution into the cell culture bag.

In the above-described aspect, the accommodation-vessel solution supply means may be provided with a solution delivery means that pumps the solution into the accommodation vessel.

Accordingly, the worker operates the solution delivery means, thereby making it possible to easily perform culture-medium replacement etc.

The above-described aspect may further include a control means that remotely controls at least one of the solution suction means and the solution delivery means.

Accordingly, the solution in the cell culture bag can be remotely replaced at arbitrary timing.

In the above-described aspect, the solution supply means may be provided with a temperature control means that controls the temperature of the culture medium.

Accordingly, it is possible to provide the culture medium at a right temperature for cell culturing, thus preventing unnecessary stress from being put on cells.

In the above-described aspect, the solution supply means may be provided with an oxygen supply means that supplies oxygen to the culture medium.

Accordingly, it is possible to provide an environment more suitable for cell growth.

According to another aspect, the present invention provides a cell culture bag that is formed of a tubular membrane material that can accommodate cells and a culture medium therein and that has openings at both ends in the longitudinal direction; the cell culture bag forming a flow passage for the culture medium from one opening toward the other opening in the longitudinal direction of the tubular membrane material; and the tubular membrane material having flexibility allowing it to be bent with the culture medium held therein, in order to bend the flow passage.

### {Advantageous Effects of Invention}

According to the present invention, in cell culturing using a cell culture bag, culture-medium replacement and cell passaging can be easily performed, thus eliminating complicated work for culture-medium replacement and cell passaging, and thus, it is possible to prevent a cell culture system from being contaminated by bacteria etc. Furthermore, because a flow-passage-like cell culture system can be formed, the cell adhesion area can be efficiently increased in a limited space.

### {Brief Description of Drawings}

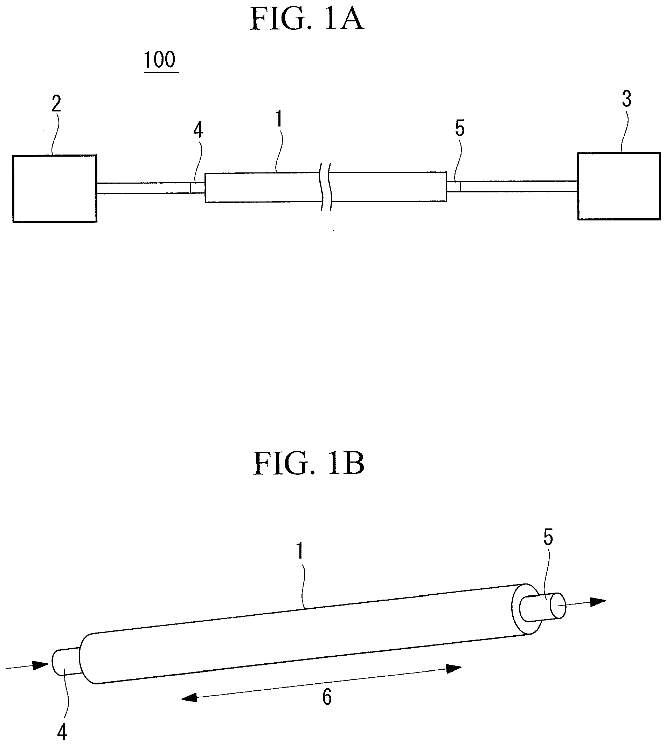

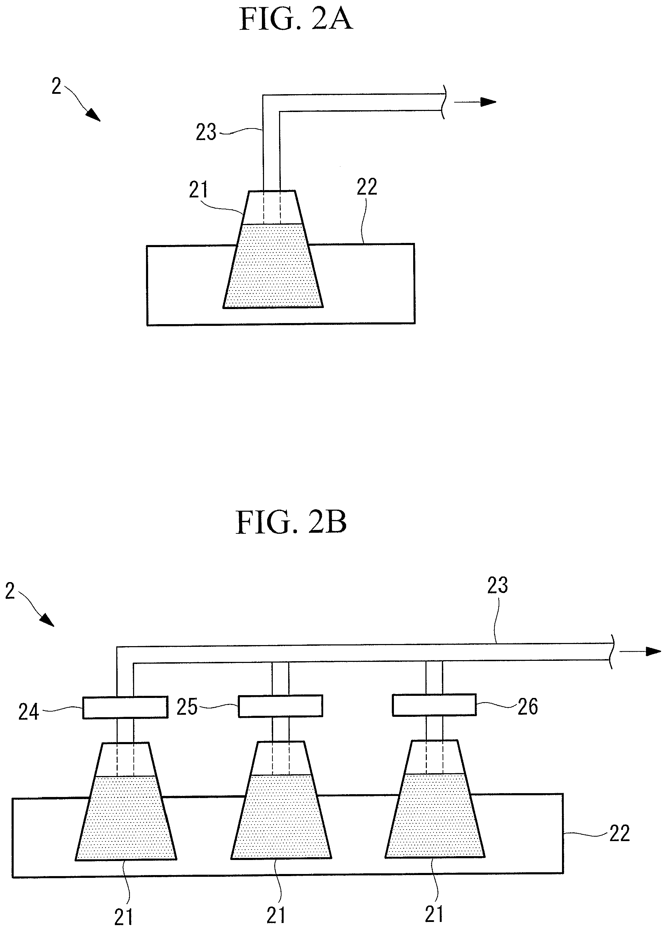

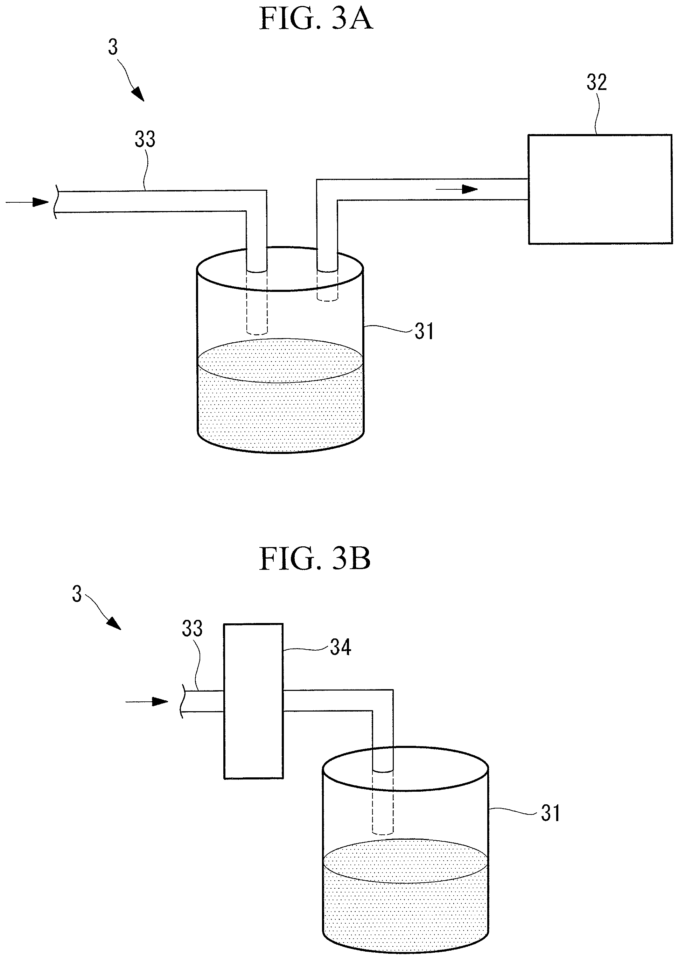

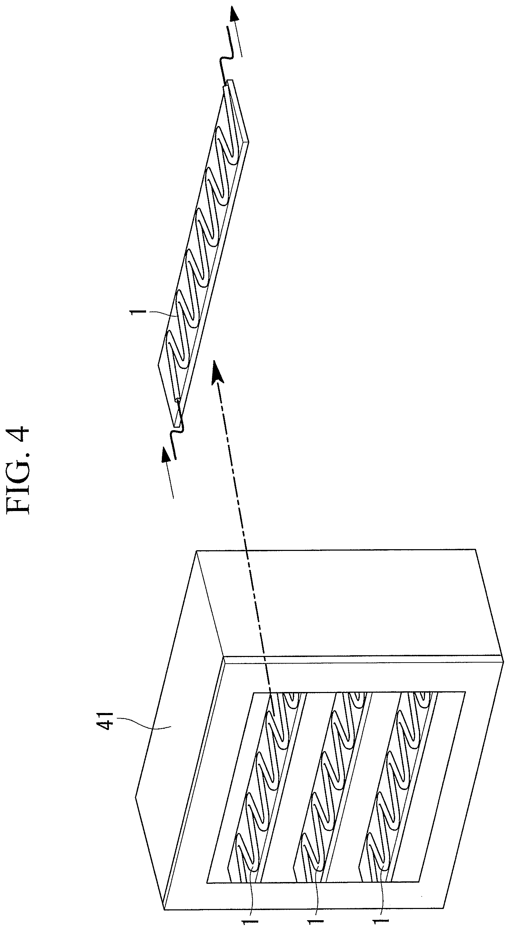

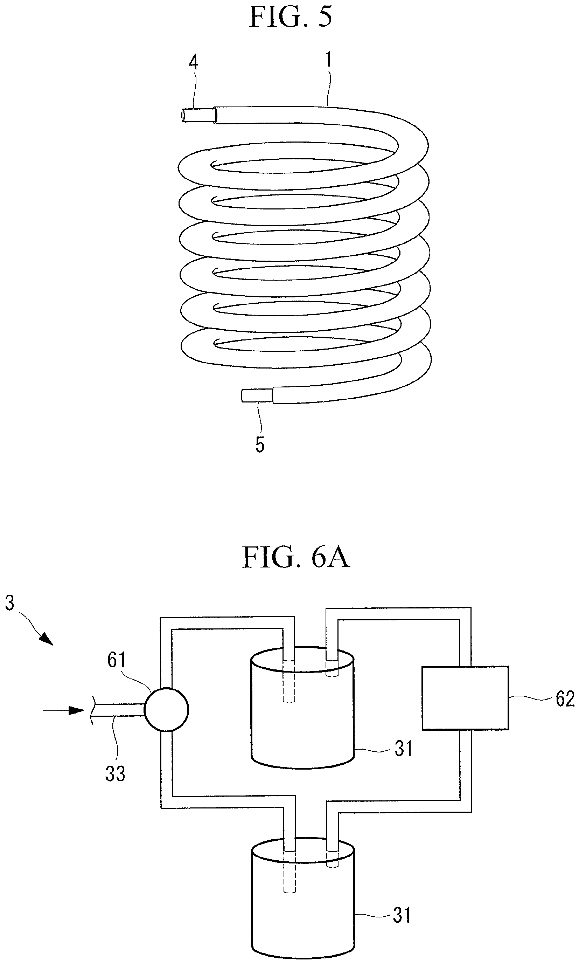

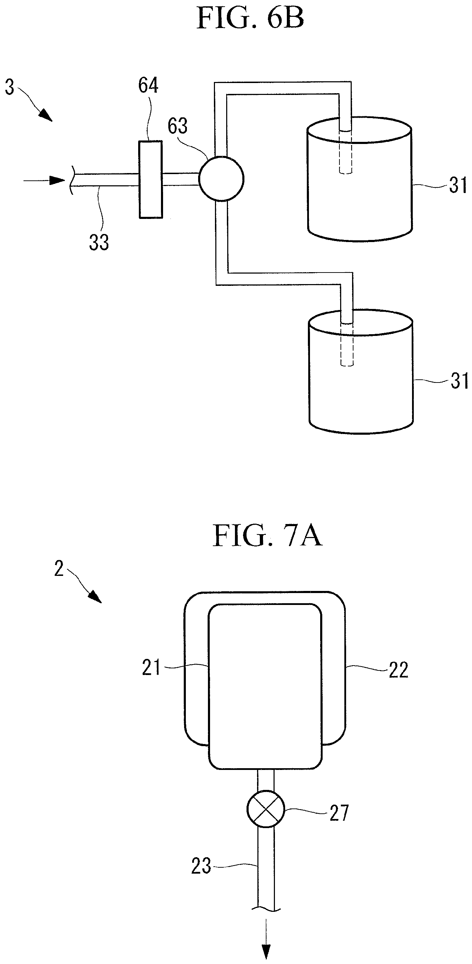

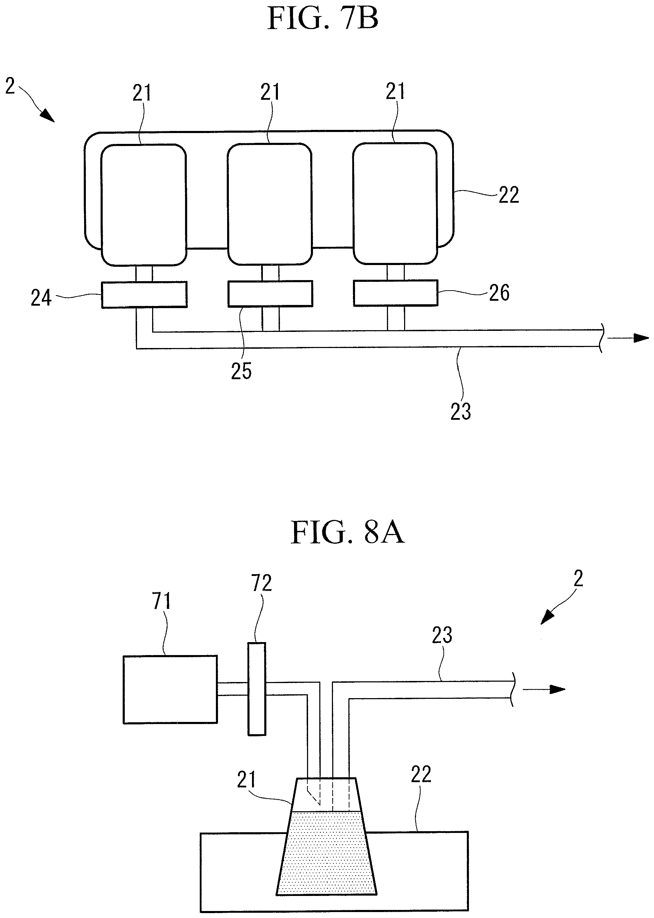

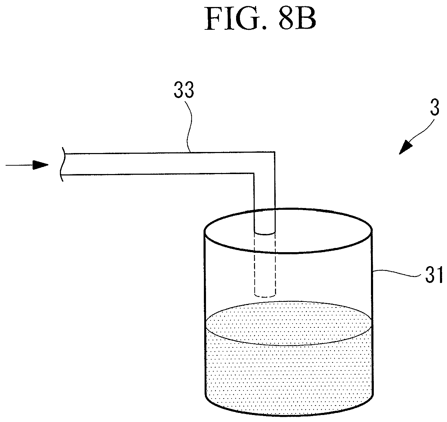

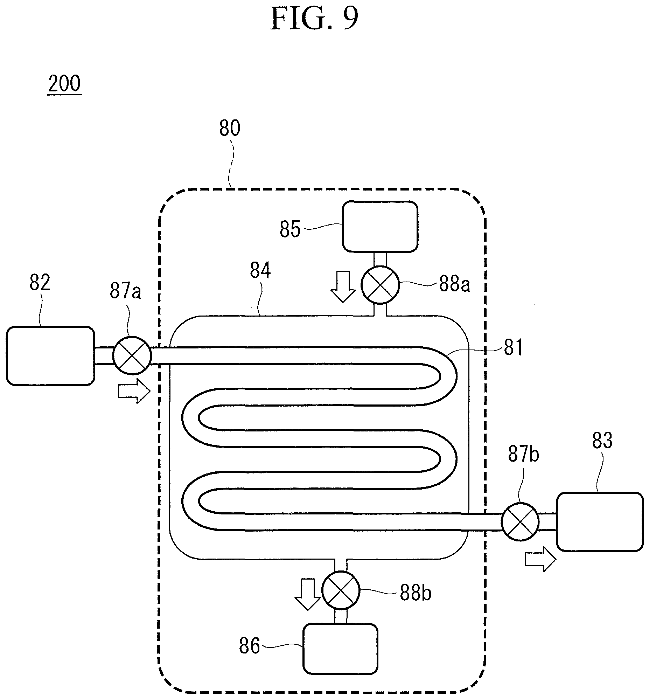

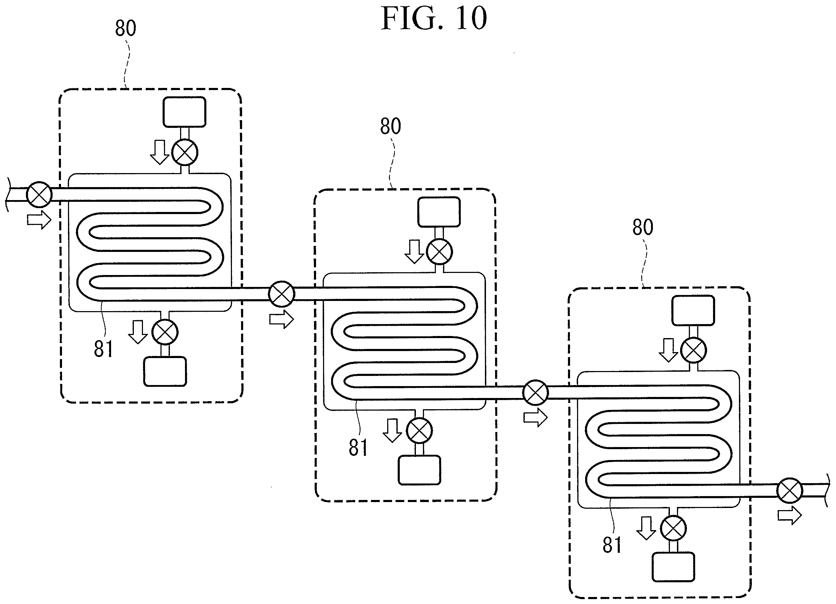

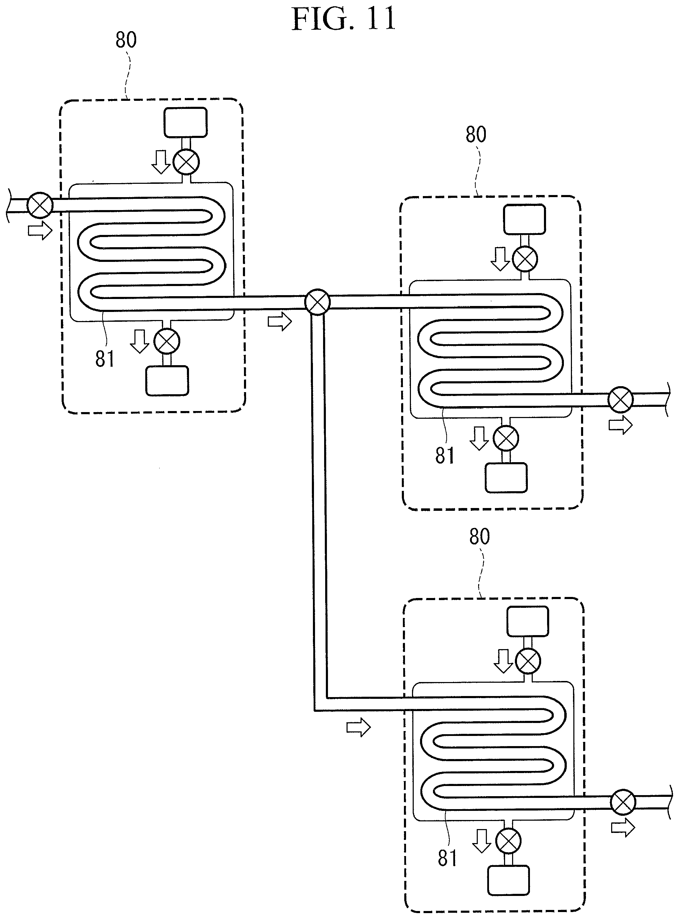

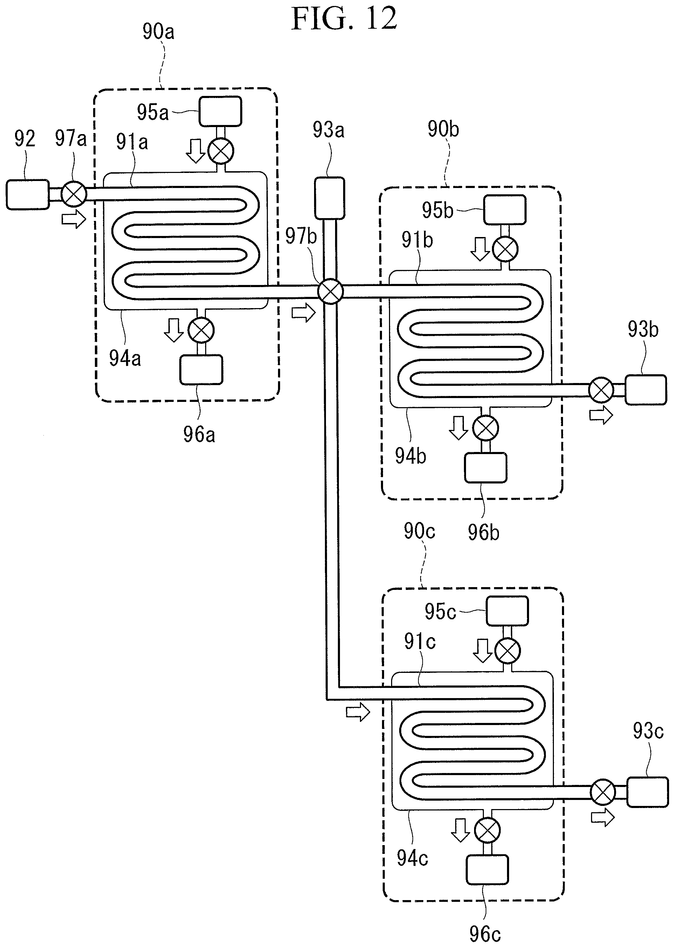

{Fig. 1A} Fig. 1A is an explanatory view showing, in outline, the configuration of a cell culture apparatus according to a first embodiment of the present invention.
{Fig. 1B} Fig. 1B is an explanatory view showing, in outline, the configuration of a cell culture bag in the cell culture apparatus shown in Fig. 1A.
{Fig. 2A} Fig. 2A is an explanatory view showing, in outline, an example configuration of a solution supply means in the cell culture apparatus shown in Fig. 1A.
{Fig. 2B} Fig. 2B is an explanatory view showing, in outline, the configuration of a modification of the solution supply means shown in Fig. 2A.
{Fig. 3A} Fig. 3A is an explanatory view showing, in outline, an example configuration of a solution discharge means in the cell culture apparatus shown in Fig. 1A.
{Fig. 3B} Fig. 3B is an explanatory view showing, in outline, the configuration of a modification of the solution discharge means shown in Fig. 3A.
{Fig. 4} Fig. 4 is an explanatory view showing an example arrangement of the cell culture bag shown in Fig. 1B.
{Fig. 5} Fig. 5 is an explanatory view showing another example arrangement of the cell culture bag shown in Fig. 1B.
{Fig. 6A} Fig. 6A is an explanatory view showing, in outline, the configuration of another modification of the solution discharge means shown in Fig. 3A.
{Fig. 6B} Fig. 6B is an explanatory view showing, in outline, the configuration of still another modification of the solution discharge means shown in Fig. 3A.
{Fig. 7A} Fig. 7A is an explanatory view showing, in outline, the configuration of another modification of the solution supply means shown in Fig. 2A.
{Fig. 7B} Fig. 7B is an explanatory view showing, in outline, the configuration of still another modification of the solution supply means shown in Fig. 2A.
{Fig. 8A} Fig. 8A is an explanatory view showing, in outline, the configuration of still another modification of the solution supply means shown in Fig. 2A.
{Fig. 8B} Fig. 8B is an explanatory view showing, in outline, the configuration of still another modification of the solution discharge means shown in Fig. 3A.
{Fig. 9} Fig. 9 is an explanatory view showing the basic configuration of a cell culture apparatus according to a second embodiment of the present invention.
{Fig. 10} Fig. 10 is an explanatory view showing, in outline, an example configuration of the cell culture apparatus according to the second embodiment of the present invention.
{Fig. 11} Fig. 11 is an explanatory view showing, in outline, another example configuration of the cell culture apparatus according to the second embodiment of the present invention.
{Fig. 12} Fig. 12 is an explanatory view showing, in outline, still another example configuration of the cell culture apparatus according to the second embodiment of the present invention.

### {Description of Embodiments}

### First Embodiment

A cell culture apparatus 100 according to a first embodiment of the present invention will be described below with reference to the drawings.

The cell culture apparatus 100 of this embodiment is an apparatus that is used to culture cells by using a cell culture bag and that has a configuration shown in Fig. 1A.

The cell culture apparatus 100 is provided with: a tube-shaped (tubular) cell culture bag 1 in which cells are cultured; a solution supply means 2 that supplies a solution, such as a culture medium, to the cell culture bag 1; and a solution discharge means 3 that discharges the solution, such as a culture medium, from the cell culture bag 1.

The cell culture bag 1 used in this apparatus 100 is a tube-shaped bag that is formed of a membrane material having high gas permeability, and has, at both ends of the tube shape, openings through which cells and a culture medium (cell culture solution) can be taken in and out. One of the openings functions as a supply port 4 and is connected to the solution supply means 2. The other opening functions as a discharge port 5 and is connected to the solution discharge means 3. This tube-shaped bag has flexibility so as to be freely bent to some extent, with a solution, such as a culture medium, held therein.

Specifically, the cell culture bag 1 is a cell culture bag that is formed of a tubular membrane material that can accommodate cells and a culture medium therein, that has openings at both ends in a longitudinal direction 6 of the tubular membrane material, that forms a flow passage for the culture medium from one opening to the other opening in the longitudinal direction 6 of the tubular membrane material, and in which the tubular membrane material has flexibility allowing it to be bent with the culture medium held therein, in order to bend the flow passage.

Fig. 2A shows an example of the solution supply means 2. The solution supply means 2 is provided with: a solution holding means 21 that holds a solution, such as a culture medium; a solution temperature control means 22 that controls the temperature of the solution; and a pipe member 23 that connects the supply port 4 of the cell culture bag 1 and the solution holding means 21.

The solution holding means 21 is formed of a vessel or a bag having an arbitrary form that can accommodate the solution, for example, and the inside thereof can be sealed.

The solution temperature control means 22 is a thermostatic bath or a heater, for example.

Fig. 3A shows an example of the solution discharge means 3. The solution discharge means 3 is provided with: a waste-liquid holding means 31 that holds the solution, such as a culture medium, discharged from the cell culture bag 1; a negative-pressure supply means 32 that functions as a solution suction means by applying a negative pressure to the waste-liquid holding means 31; and a pipe member 33 that connects the discharge port 5 of the cell culture bag 1 and the waste-liquid holding means 31.

The waste-liquid holding means 31 is formed of a vessel or a bag having an arbitrary form that can hold the solution, for example, and the inside thereof can be sealed.

The negative-pressure supply means 32 is formed of: a pipe that communicates with the inside of the waste-liquid holding means 31; and a gas discharge pump that discharges gas in the waste-liquid holding means 31 via the pipe, for example.

The tube-shaped cell-culture bag 1 is disposed in an incubator that can maintain an appropriate culture environment for cells. At this time, because the tube-shaped cell-culture bag 1 has flexibility allowing it to be bent in a desired direction with the culture medium held therein, as shown in Fig. 4, for example, the tube-shaped cell-culture bag 1 can be disposed on a rack in an incubator 41 so as to form a flow passage in the horizontal direction.

Alternatively, for example, as shown in Fig. 5, the tube-shaped cell-culture bag 1 may be disposed in a spiral manner. In order to maintain breathability required for cell growth in the cell culture bag 1, the tube-shaped cell-culture bag 1 may be disposed so as to secure contact surfaces with an air space in the incubator.

The solution supply means 2 and the solution discharge means 3 can be disposed at arbitrary locations where the solution, such as a culture medium, can be supplied to and discharged from the cell culture bag 1.

Next, an example procedure for replacing the culture medium by using the cell culture apparatus 100 of this embodiment will be described.

A user of this apparatus 100 first prepares the cell culture bag 1, which is filled with cells to be cultured (adherent cells) and a culture medium (cell culture solution), and disposes the cell culture bag 1 in the incubator. Next, the user connects the supply port 4 of the cell culture bag 1 to the solution supply means 2 and connects the discharge port 5 of the cell culture bag 1 to the solution discharge means 3. The cells can be cultured in this state.

The user applies, at arbitrary timing, a negative pressure to the waste-liquid holding means 31 by means of the negative-pressure supply means 32 of the solution discharge means 3. Accordingly, the culture medium in the cell culture bag 1 passes through the pipe member 33 of the solution discharge means 3 and is discharged to the waste-liquid holding means 31, and, at the same time, at an upstream side of the flow passage, the culture medium is supplied from the solution holding means 21 of the solution supply means 2 to the cell culture bag 1 via the pipe member 23 of the solution supply means 2. The user manipulates the negative-pressure supply means 32 at arbitrary timing, thereby making it possible to control discharge and supply of the culture medium.

Alternatively, it is also possible to adjust the amount of negative pressure to be applied by the negative-pressure supply means 32 and to keep discharging the culture medium from the cell culture bag 1 at a constant discharge rate. In this case, the culture medium is supplied from the solution supply means 2 to the cell culture bag 1 at the same rate.

In this embodiment, although an example case in which adherent cells are cultured has been illustrated, the present invention can also be applied to culturing of floating cells by making the flow rate of a solution flowing in the cell culture bag 1 sufficiently slow. Specifically, because floating cells, while being cultured, exist on the bottom surface of the cell culture bag 1 due to the force of gravity, the present invention can also be applied to culturing of floating cells as long as the flow rate is set such that the cells are not moved much in the flow rate direction.

The present invention can be applied to culturing of floating cells by disposing, at the discharge port 5, a filter that does not allow the cells to pass therethrough.

In this embodiment, as the solution suction means, instead of the negative-pressure supply means 32, it is also possible to adopt a liquid feeding pump 34 (a peristaltic pump, a tubing pump, or the like), as shown in Fig. 3B. In this case, the liquid feeding pump 34 is disposed in the pipe member 33, which connects the discharge port 5 of the cell culture bag 1 and the waste-liquid holding means 31.

In this embodiment, it is also possible to provide a control means that can remotely operate the solution suction means, such as the negative-pressure supply means 32 and the liquid feeding pump 34. The remote operation in this case can be performed in a wired or wireless manner. The user operates the control means, thereby making it possible to remotely replace the culture medium, and thus it is possible to save the user the trouble of walking into a work space, as well as the costs thereof, and to more reliably prevent the cell culture system from being contaminated by bacteria etc.

In this embodiment, the solution supply means 2 may be provided with an oxygen supply means (not shown) that supplies oxygen to the culture medium to be supplied to the cell culture bag 1. An example of the oxygen supply means is a means for feeding oxygen or gas containing oxygen to the culture medium in the solution holding means 21 through bubbling or the like. Accordingly, even when the contact surface between the cell culture bag 1 and the air space is not sufficiently secured, cell culturing can be performed; furthermore, a cell culture bag 1 that is formed of a material having low gas permeability can also be used.

In this embodiment, as shown in Fig. 2B, the solution supply means 2 may be provided with a plurality of solution holding means 21 that hold a plurality of different types of solutions, respectively. In this case, the solution supply means 2 may be provided with a flow-passage selecting means for selecting the solution to be supplied to the cell culture bag 1, and the flow-passage selecting means may have a function of selecting (switching) among the flow passages extending from the plurality of solution holding means 21.

For example, in the example shown in Fig. 2B, the solution supply means 2 is provided with, in the flow passages from the respective solution holding means 21, flow-passage opening/closing means 24, 25, and 26 for opening and closing the corresponding flow passages. By controlling the flow-passage opening/closing means 24, 25, and 26, a desired solution can be supplied to the cell culture bag 1. Here, it is also possible to provide a control means that remotely operates the flow-passage opening/closing means (flow-passage selecting means) 24, 25, and 26. The remote operation in this case can be performed in a wired or wireless manner.

The plurality of solution holding means 21 may respectively hold, for example, a culture medium, a cleaning solution, and a cell detachment solution (a trypsin-containing solution or the like).

In this embodiment, the solution discharge means 3 may be provided with a plurality of waste-liquid holding means 31, as shown in Fig. 6A. In this case, a negative-pressure supply means 62 can supply a negative pressure to the respective waste-liquid holding means 31. Furthermore, the pipe member 33 connected from the discharge port 5 of the cell culture bag 1 is split at a middle point and is connected to the respective waste-liquid holding means 31. In this case, the solution discharge means 3 may be provided with a discharge flow-passage selecting means 61 (for example, a switching valve) for selecting the waste-liquid holding means 31, and the discharge flow-passage selecting means 61 may have a function of selecting (switching) between the flow passages to the plurality of waste-liquid holding means 31.

Furthermore, when a liquid feeding pump is used as the solution suction means, it is also possible to provide a plurality of waste-liquid holding means 31, as shown in Fig. 6B. In this case, a liquid feeding pump 64 (a peristaltic pump, a tubing pump, or the like) may be disposed in the pipe member 33 at an upstream side of the branch point, and a discharge flow-passage selecting means 63 may select (switch) between the flow passages to the plurality of waste-liquid holding means 31. Alternatively, liquid feeding pumps may be disposed in the respective pipe members 33 that are located at downstream sides of the branch point, and the liquid feeding pump to be actuated may be selected, thereby selecting the waste-liquid holding means 31 to be used.

Here, it is also possible to provide a control means that can remotely operate the discharge flow-passage selecting means 61, 63 in a wired or wireless manner.

In this embodiment, in the solution supply means 2, a solution holding means 21 shown in Fig. 7A may be disposed at a position higher in the direction of gravitational force than the cell culture bag 1, and the solution may be gravitationally supplied to the cell culture bag 1. At this time, the pipe member 23 is provided with a supply-rate adjusting means 27 (for example, a flow-rate control valve), so that the rate of supply of the solution to the cell culture bag 1 can be adjusted. A control means that can remotely operate the supply-rate adjusting means 27 may be provided. The remote operation in this case can be performed in a wired or wireless manner. As shown in Fig. 7A, the temperature of the solution in the solution holding means 21 may be controlled by the solution temperature control means 22.

Furthermore, in this form, the negative-pressure supply means 32 need not be provided as long as the solution discharge means 3 is disposed at a position lower in the direction of gravitational force than the cell culture bag 1. The solution, such as a culture medium, discharged from the cell culture bag 1 may be gravitationally collected in the waste-liquid holding means 31.

As shown in Fig. 7B, the solution supply means 2 may be provided with a plurality of solution holding means 21 that hold a plurality of different types of solutions, respectively. In this case, the solution supply means 2 may be provided with a flow-passage selecting means for selecting the solution to be supplied to the cell culture bag 1, and the flow-passage selecting means may have a function of selecting (switching) among the flow passages extending from the plurality of solution holding means 21.

For example, in the example shown in Fig. 7B, the solution supply means 2 is provided with, in the flow passages extending from the respective solution holding means 21, flow-passage opening/closing means 24, 25, and 26 for opening and closing the corresponding flow passages. By controlling the opening and closing of the flow-passage opening/closing means 24, 25, and 26, a desired solution can be supplied to the cell culture bag 1. The flow-passage opening/closing means 24, 25, and 26 each function as the supply-rate adjusting means, so that the rate of supply of the solution to the cell culture bag 1 can be adjusted. Here, it is also possible to provide a control means that can remotely operate the flow-passage opening/closing means (flow-passage selecting means) 24, 25, and 26. The remote operation in this case can be performed in a wired or wireless manner.

The plurality of solution holding means 21 may respectively hold, for example, a culture medium, a cleaning solution, and a cell detachment solution (trypsin or the like).

In this embodiment, although a form in which the solution is supplied to and discharged from the cell culture bag 1 by a negative pressure supplied by the negative-pressure supply means 32 has been illustrated, it is also possible to adopt a positive-pressure applying means 71 that applies a positive pressure to the solution holding means 21 of the solution supply means 2, without using the negative-pressure supply means 32.

As the positive-pressure applying means 71, for example, a means (a gas supply pump or the like) for pumping gas into the solution holding means 21, shown in Fig. 8A, can be used, and, gas may be pumped into the solution holding means 21 to apply a positive pressure to the inside of the solution holding means 21, thus pumping out the solution held in the solution holding means 21 via the pipe member 23. It is preferred that gas to be pumped into the solution holding means 21 from the positive-pressure applying means 71 be sterilized, and, for example, gas may be pumped through a filter 72. Alternatively, without adopting the filter 72, sterilized gas may be pumped.

The solution pumped out to the pipe member 23 is supplied to the cell culture bag 1 from the supply port 4, and, at the same time, a substantially equivalent amount of the solution is discharged from the discharge port 5 of the cell culture bag 1 and is pumped out to the solution discharge means 3. In this case, for example, as shown in Fig. 8B, the solution discharge means 3 is formed of the waste-liquid holding means 31 and the pipe member 33, which is connected to the discharge port 5 of the cell culture bag 1, and the solution discharged from the cell culture bag 1 is discharged to the waste-liquid holding means 31 via the pipe member 33 and is held therein.

Furthermore, instead of a form in which the positive-pressure applying means 71 applies a positive pressure to the solution holding means 21, it is also possible to provide a liquid feeding pump (a peristaltic pump, a tubing pump, or the like) in the pipe member 23 to supply the solution to the cell culture bag 1.

In this embodiment, the supply-rate adjusting means 27 and the positive-pressure applying means 71 function as a solution delivery means.

### Second Embodiment

Next, a cell culture apparatus 200 according to a second embodiment of the present invention will be described with reference to the drawings.

The cell culture apparatus 200 of this embodiment is an apparatus that is used to culture cells by using a cell culture bag and that has the basic configuration shown in Fig. 9.

The cell culture apparatus 200 is provided with: a cell culture unit 80 that includes a tube-shaped (tubular) cell culture bag 81 in which cells are cultured; a solution supply means 82 that supplies a solution, such as a culture medium, to the cell culture bag 81; and a solution discharge means 83 that discharges the solution, such as a culture medium, from the cell culture bag 81.

The cell culture unit 80 is provided with: the tube-shaped (tubular) cell culture bag 81, in which cells are cultured; an accommodation vessel 84 that accommodates at least part of the cell culture bag 81 and that can hold a solution therein; an accommodation-vessel solution supply means 85 that supplies the solution, such as a culture medium, to the accommodation vessel 84; and an accommodation-vessel solution discharge means 86 that discharges the solution, such as a culture medium, from the accommodation vessel 84.

The accommodation-vessel solution supply means 85 is connected to a supply port of the accommodation vessel 84 via a pipe member.

The accommodation-vessel solution discharge means 86 is connected to a discharge port of the accommodation vessel 84 via a pipe member.

The accommodation vessel 84 used in this embodiment can be any vessel as long as it accommodates at least part of the cell culture bag 81 and can hold the solution therein, and, for example, a sac-like (bag-like) vessel can be used.

At least part of the tube-shaped (tubular) cell culture bag 81 used in this apparatus 200 is formed of a porous membrane. The porous membrane is formed from a membrane material that has pores through which cells cannot pass but components required for cell growth, such as oxygen and nutritional substances, and carbon dioxide and waste products, which are discharged from cells, can freely pass. The pore diameter of the porous membrane is, for example, 0.5 µm to 5 µm.

The tube-shaped cell culture bag 81 has, at both ends, openings through which cells and a culture medium (cell culture solution) can be taken in and out. One of the openings functions as a supply port and is connected to the solution supply means 82. The other opening functions as a discharge port and is connected to the solution discharge means 83.

The solution supply means 82 and the solution discharge means 83 in this embodiment may be the same as the solution supply means 2 and the solution discharge means 3 in the first embodiment, respectively. Furthermore, the accommodation-vessel solution supply means 85 and the accommodation-vessel solution discharge means 86 in this embodiment may be the same as the solution supply means 2 and the solution discharge means 3 in the first embodiment, respectively.

Valves 87a and 87b are installed at the supply port and the discharge port of the tube-shaped cell culture bag 81, respectively. The valves 87a and 87b function as gates capable of opening and closing portions of the flow passage on which they are installed.

A valve 88a is installed in the pipe member that connects the accommodation-vessel solution supply means 85 and the supply port of the accommodation vessel 84. A valve 88b is installed in the pipe member that connects the accommodation-vessel solution discharge means 86 and the discharge port of the accommodation vessel 84. The valves 88a and 88b function as gates capable of opening and closing portions of the flow passage on which they are installed.

The cell culture apparatus 200 of this embodiment has the configuration shown in Fig. 9, as the basic configuration, and it is also possible to couple a plurality of cell culture units 80 to each other, thereby extending the flow passage formed by the tube-shaped (tubular) cell culture bags 81. For example, it is possible to couple the plurality of cell culture bags 81 in series, as shown in Fig. 10, or to couple some of the cell culture bags 81 in parallel, as shown in Fig. 11. In this way, the flow passage is extended, thereby making it possible to expand the cell contact surface, and cells are passaged to the extended flow passage, thereby making it possible to increase the number of cells.

An example method for passaging cells by using the cell culture apparatus 200 of this embodiment will be described with reference to Fig. 12.

First, cells and a culture medium are supplied from a solution supply means 92 to a tube-shaped cell culture bag 91a. To the single cell culture bag 91a at a previous stage, two cell culture bags 91b and 91c at a subsequent stage are connected in parallel via a valve 97b. The valve 97b is disposed at a branch point of the flow passage and is capable of selectively opening and closing any one of flow passages. At this time, the valve 97b is in a state in which it opens a flow passage extending toward a solution discharge means 93a. An accommodation vessel 94a is supplied with a culture medium from an accommodation-vessel solution supply means 95a and is filled with the culture medium. In this state, the cells grow and proliferate in the cell culture bag 91a. At this time, components required for cell growth and waste products discharged from the cells can be replaced with the culture medium in the accommodation vessel 94a through a porous membrane of the cell culture bag 91a.

In a state in which the inside of the cell culture bag 91a is filled with the proliferated cells (a confluent state), the culture medium in the cell culture bag 91a is discharged by the solution discharge means 93a, and the culture medium in the accommodation vessel 94a is discharged by an accommodation-vessel solution discharge means 96a. Next, a cell detachment solution (trypsin-containing solution or the like) is supplied to the accommodation vessel 94a by the accommodation-vessel solution supply means 95a. The cell detachment solution in the accommodation vessel 94a passes through the cell culture bag 91a, thus entering the inside thereof, and the cells that have adhered to the inside of the cell culture bag 91a are detached.

Next, flow passages extending toward a cell culture unit 90b and a cell culture unit 90c are opened by means of the valve 97b, and the culture medium is supplied to a cell culture unit 90a by the solution supply means 92. The supplied culture medium entrains the cells detached in the cell culture bag 91a and is delivered to the cell culture unit 90b and the cell culture unit 90c. The cells in the culture medium delivered to the respective cell culture units 90b and 90c adhere to inner surfaces of the tube-shaped cell culture bags 91b and 91c in the respective units 90b and 90c and start proliferating. Here, accommodation vessels 94b and 94c that accommodate the cell culture bags 91b and 91c, respectively, are supplied with the culture medium from accommodation-vessel solution supply means 95b and 95c, respectively, and are filled.

In this way, according to this embodiment, by increasing the number of the cell culture units 90a, 90b, and 90c, the area of the flow passage can be increased. Therefore, it is possible to arbitrarily increase the cell adhesion area in accordance with cell proliferation and to easily perform cell passaging.

In this embodiment, a case in which the plurality of cell culture units 90a, 90b, and 90c are connected in advance has been illustrated; however, when cells in a single cell culture unit become confluent, a new cell culture unit may be connected thereto, thus expanding the cell adhesion area. In this case, according to the way in which a new cell culture unit is connected, the flow passage may be formed into an arbitrary shape, e.g., a serial shape or a parallel shape, as shown in Figs. 9 and 10, or a shape with a combination of serial and parallel shapes.

In this embodiment, it is also possible to provide a control means that can remotely operate the solution supply means, the solution discharge means, the accommodation-vessel solution supply means, the accommodation-vessel solution discharge means, and/or the respective valves, in a wired or wireless manner. In this case, it is preferred to provide a monitoring system (not shown). By doing so, while performing monitoring with the monitoring system, a worker remotely operates, as appropriate, the solution supply means, the solution discharge means, the accommodation-vessel solution supply means, the accommodation-vessel solution discharge means, and/or the respective valves, thus making it possible to remotely perform the culture-medium replacement and the cell passaging work, and it is possible to simplify the working process and to prevent contamination of the cell culture system caused by the work.

A PC can be used as an example of the control means of the present invention, and the PC can perform control performed by the control means in the above-described embodiments. Specifically, the control means is, for example, a PC that has a central processing unit (CPU) and a memory, and, the CPU executes a control program stored in the memory, thereby realizing the functions of the control means.

### {Reference Signs List}

1, 81, 91a, 91b, 91c cell culture bag
2, 82, 92 solution supply means
3, 83, 93a, 93b, 93c solution discharge means
4 supply port (opening)
5 discharge port (opening)
21 solution holding means
22 solution temperature control means
23, 33 pipe member
24, 25, 26 flow-passage opening/closing means (flow-passage selecting means)
27 supply-rate adjusting means (solution delivery means)
31 waste-liquid holding means
32, 62 negative-pressure supply means (solution suction means)
34, 64 liquid feeding pump
41 incubator
61, 63 discharge flow-passage selecting means
71 positive-pressure applying means (solution delivery means)
72 filter
80, 90a, 90b, 90c cell culture unit
84, 94a, 94b, 94c accommodation vessel
85, 95a, 95b, 95c accommodation-vessel solution supply means
86, 96a, 96b, 96c accommodation-vessel solution discharge means
87a, 87b, 88a, 88b, 97a, 97b valve
100, 200 cell culture apparatus

## Claims

1. A cell culture apparatus comprising:
a cell culture bag that is formed of a tubular membrane material that can accommodate cells and a culture medium therein and that has openings at both ends in the longitudinal direction, and that forms a flow passage for the culture medium, from one opening toward the other opening in the longitudinal direction of the tubular membrane material;
a solution supply means that is connected to the one opening and that supplies a solution to the inside of the cell culture bag; and
a solution discharge means that is connected to the other opening and that discharges the solution from the inside of the cell culture bag.

2. A cell culture apparatus according to claim 1, further comprising:
an accommodation vessel that accommodates at least part of the cell culture bag and that can hold the solution therein;
an accommodation-vessel solution supply means that supplies the solution to the accommodation vessel; and
an accommodation-vessel solution discharge means that discharges the solution from the accommodation vessel,
wherein the at least part of the cell culture bag is formed of a porous membrane.

3. A cell culture apparatus according to claim 1 or 2, wherein the solution discharge means is provided with a solution suction means that suctions the solution from the inside of the cell culture bag.

4. A cell culture apparatus according to claim 2, wherein the accommodation-vessel solution discharge means is provided with a solution suction means that suctions the solution from the inside of the accommodation vessel.

5. A cell culture apparatus according to one of claims 1 to 4, wherein the solution supply means is provided with a solution delivery means that pumps the solution into the cell culture bag.

6. A cell culture apparatus according to claim 2, wherein the accommodation-vessel solution supply means is provided with a solution delivery means that pumps the solution into the accommodation vessel.

7. A cell culture apparatus according to one of claim 3 or 4, further comprising a control means that remotely controls the solution suction means.

8. A cell culture apparatus according to one of claim 5 or 6, further comprising a control means that remotely controls the solution delivery means.

9. A cell culture bag that is formed of a tubular membrane material that can accommodate cells and a culture medium therein and that has openings at both ends in the longitudinal direction;
the cell culture bag forming a flow passage for the culture medium from one opening toward the other opening in the longitudinal direction of the tubular membrane material; and
the tubular membrane material having flexibility allowing it to be bent with the culture medium held therein, in order to bend the flow passage.
